(19) Europäisches Patentamt
European Patent Office
Office européen des brevets

(11) Veröffentlichungsnummer: **0 087 679**
**B1**

(12) # EUROPÄISCHE PATENTSCHRIFT

(45) Veröffentlichungstag der Patentschrift: **22.06.88**

(51) Int. Cl.[4]: **C 07 D 319/06**, C 09 K 19/30

(21) Anmeldenummer: **83101466.7**

(22) Anmeldetag: **16.02.83**

(54) **1,3-Dioxane.**

(30) Priorität: **27.02.82 DE 3207114**

(43) Veröffentlichungstag der Anmeldung:
**07.09.83 Patentblatt 83/36**

(45) Bekanntmachung des Hinweises auf die Patenterteilung:
**22.06.88 Patentblatt 88/25**

(84) Benannte Vertragsstaaten:
**CH DE FR GB LI**

(56) Entgegenhaltungen:
**FR - A - 2 444 071**
**FR - A - 2 511 696**
**FR - A - 2 516 082**
**US - A - 4 323 473**
**US - A - 4 325 830**
**US - A - 4 344 856**

**MOLECULAR CRYSTALS & LIQUID CRYSTALS, Band 63, Nr. 1-4, 1981, Seiten 129-144, Gordon and Breach Science Publishers, Inc., New York, US; D. DEMUS et al.: "Synthesis and properties of new liquid crystalline materials"**

(73) Patentinhaber: **Merck Patent Gesellschaft mit beschränkter Haftung, Frankfurter Strasse 250, D-6100 Darmstadt (DE)**

(72) Erfinder: **Römer, Michael, Dr. Dipl.-Ing., Im Grossen Garten 18, D-6054 Rodgau 2 (DE)**
Erfinder: **Krause, Joachim, Dr., Samuel Morse Strasse 14, D-6110 Dieburg (DE)**
Erfinder: **Weber, Georg, Wilhelm-Leuschner-Strasse 38, D-6106 Erzhausen (DE)**

**EP 0 087 679 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue 1,3-Dioxane der Formel I

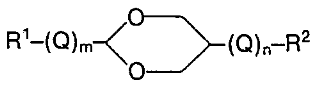

(I)

worin

$R^1$ H, Alkyl, Alkoxy, Alkanoyloxy oder Alkoxycarbonyl mit jeweils 1 bis 10 C-Atomen, F, Cl, Br, $CF_3$ oder CN,

$R^2$ Alkyl oder Alkoxy mit jeweils 1–10 C-Atomen, die Reste Q, die gleich oder verschieden sein können, 1,4-Phenylen- oder 1,4-Cyclohexylenreste und die Parameter m und n jeweils 0, 1 oder 2, ihre Summe (m + n) jedoch 2 oder 3 bedeuten, mit der Massgabe, dass im Falle m = n = 1 mindestens einer der beiden Reste Q ein 1,4-Cyclohexylenrest ist.

Diese Verbindungen können wie ähnliche, z.B. aus der DE-OS 2 944 905 bekannte Verbindungen als Komponenten flüssigkristalliner Dielektrika verwendet werden, insbesondere für Anzeigeelemente, die auf dem Prinzip der verdrillten Zelle beruhen.

Die bisher für diesen Zweck eingesetzten Substanzen besitzen sämtlich gewisse Nachteile, beispielsweise zu hohe Schmelzpunkte, zu niedrige Klärpunkte, zu geringe Stabilität gegenüber der Einwirkung von Wärme, Licht oder elektrischen Feldern, zu hohe Temperaturabhängigkeit der Schwellenspannung.

Aus Mol. Cryst. Liqu. Cryst. 1981, Vol. 63, pp. 129–144 sind 2-(p-Cyanphenyl)-5-(p-alkylphenyl)-1,3-dioxane und p-Alkyl- bzw. p-Alkoxybenzoesäure-p-(5-alkyl-1,3-dioxan-2-yl)-phenylester als Komponenten flüssigkristalliner Dielektrika bekannt. Die Verbindungen der Formel I sind jedoch wesentlich besser geeignet, in Dielektrika die Klärpunkte zu erhöhen bei nahezu unveränderter oder nur unwesentlich erhöhter Viskosität.

Der Erfindung lag die Aufgabe zugrunde, neue flüssigkristalline Verbindungen aufzufinden, die als Komponenten flüssigkristalliner Dielektrika geeignet sind, insbesondere für nematische Phasen mit hoher dielektrischer Anisotropie, und die die Nachteile der bekannten Verbindungen nicht oder nur in geringerem Masse zeigen. Diese Aufgabe wurde durch die Bereitstellung der neuen Verbindungen der Formel I gelöst.

Es wurde gefunden, dass die Verbindungen der Formel I vorzüglich als Komponenten flüssigkristalliner Dielektrika geeignet sind. Insbesondere sind mit ihrer Hilfe flüssigkristalline Phasen mit weiten nematischen Bereichen geringer Temperaturabhängigkeit der Schwellenspannung und/oder kleiner optischer Anisotropie erhältlich. Die neuen Verbindungen zeigen ausserdem eine gute Löslichkeit für andere Komponenten derartiger Dielektrika und, besonders diejenigen unter ihnen, worin der Rest $R^1$ eine CN-Gruppe bedeutet, hohe positive dielektrische Anisotropie.

Die Verbindungen der Formel I sind in reinem Zustand farblos und bilden flüssigkristalline Mesophasen in einem für die elektrooptische Verwendung günstig gelegenen Temperaturbereich.

Gegenstand der Erfindung sind somit die Verbindungen der Formel I sowie ein Verfahren zu ihrer Herstellung, das dadurch gekennzeichnet ist, dass man einen Aldehyd der Formel II

$$R^1\text{–}(Q)_m\text{–}CHO \qquad (II)$$

worin

$R^1$, Q und m die angegebene Bedeutung haben oder eines seiner funktionellen Derivate mit einem Diol der Formel III

$$(HOCH)_2)_2CH\text{–}(Q)_n\text{–}R^2 \qquad (III)$$

worin

$R^2$, Q und n die angegebene Bedeutung haben, umsetzt.

Weiterhin sind Gegenstand der Erfindung die Verwendung der Verbindungen der Formel I als Komponenten flüssigkristalliner Dielektrika, flüssigkristalline Dielektrika mit einem Gehalt an mindestens einer Verbindung der Formel I sowie elektrooptische Anzeigeelemente, die derartige Dielektrika enthalten.

Vor- und nachstehend haben $R^1$, $R^2$, Q, m und n die angegebene Bedeutung, sofern nicht ausdrücklich etwas anderes vermerkt ist.

Die Verbindungen der Formel I umschliessen im einzelnen Verbindungen folgender Teilformeln:

| | |
|---|---|
| $R^1$–Ph–Ph–Dio–$R^2$ | Ia |
| $R^1$–Ph–Cy–Dio–$R^2$ | Ib |
| $R^1$–Cy–Ph–Dio–$R^2$ | Ic |
| $R^1$–Cy–Cy–Dio–$R^2$ | Id |
| $R^1$–Ph–Dio–Cy–$R^2$ | Ie |
| $R^1$–Cy–Dio–Ph–$R^2$ | If |
| $R^1$–Cy–Dio–Cy–$R^2$ | Ig |
| $R^1$–Dio–Ph–Ph–$R^2$ | Ih |
| $R^1$–Dio–Ph–Cy–$R^2$ | Ii |
| $R^1$–Dio–Cy–Ph–$R^2$ | Ij |
| $R^1$–Dio–Cy–Cy–$R^2$ | Ik |
| $R^1$–Ph–Ph–Dio–Ph–$R^2$ | Il |
| $R^1$–Ph–Ph–Dio–Cy–$R^2$ | Im |
| $R^1$–Ph–Cy–Dio–Ph–$R^2$ | In |
| $R^1$–Ph–Cy–Dio–Cy–$R^2$ | Io |
| $R^1$–Cy–Ph–Dio–Ph–$R^2$ | Ip |
| $R^1$–Cy–Ph–Dio–Cy–$R^2$ | Iq |
| $R^1$–Cy–Cy–Dio–Ph–$R^2$ | Ir |
| $R^1$–Cy–Cy–Dio–Cy–$R^2$ | Is |
| $R^1$–Ph–Dio–Ph–Ph–$R^2$ | It |
| $R^1$–Ph–Dio–Ph–Cy–$R^2$ | Iu |
| $R^1$–Ph–Dio–Cy–Ph–$R^2$ | Iv |
| $R^1$–Ph–Dio–Cy–Cy–$R^2$ | Iw |
| $R^1$–Cy–Dio–Ph–Ph–$R^2$ | Ix |
| $R^1$–Cy–Dio–Ph–Cy–$R^2$ | Iy |
| $R^1$–Cy–Dio–Cy–Ph–$R^2$ | Iz |
| $R^1$–Cy–Dio–Cy–Cy–$R^2$ | Iaa |

Verbindungen der Formeln Ia, Ic und Iu, ferner diejenigen der Formeln Ie, Il, Im, Ip, Iq, It, Iv und Iw, die die Teilgruppe –Ph–Dio– enthalten, sind bevorzugt.

Bevorzugt sind ferner Verbindungen der Formel I sowie Ia bis Iaa, worin der Rest $R^1$ H, Alkanoyloxy

oder Alkoxycarbonyl mit jeweils 1 bis 10 C-Atomen, F, Cl, Br, CN oder $CF_3$ bedeutet.

In den Verbindungen der Formeln Ia bis Iaa haben die Alkyl-, Alkoxy-, Alkanoyloxy- oder Alkoxycarbonylgruppen vorzugsweise jeweils 1–6, insbesondere 3, 4, 5 oder 6 C-Atome. Der Rest $R^1$ ist vorzugsweise Alkyl oder CN, der Rest $R^2$ ist vorzugsweise Alkyl.

In den Verbindungen der Formeln I sowie Ia bis Iaa sind diejenigen Stereoisomeren bevorzugt, in denen die beiden Substituenten am Dioxandiylrest und an den Cyclohexylenresten jeweisl in trans-Stellung zueinander stehen. In den Verbindungen der Formel I sowie Ia bis Iaa sind die Alkyl-, Alkoxy-, Alkanoyloxy und Alkoxycarbonylgruppen vorzugsweise geradkettig.

Dementsprechend bedeutet Alkyl vorzugsweise Methyl, Ethyl, n-Propyl, n-Butyl, n-Pentyl, n-Hexyl, n-Heptyl, n-Octyl, n-Nonyl oder n-Decyl; Alkoxy bedeutet vorzugsweise Methoxy, Ethoxy, n-Propyloxy, n-Butyloxy, n-Pentyloxy, n-Hexyloxy, n-Heptyloxy, n-Octyloxy, n-Nonyloxy oder n-Decyloxy; Alkanoyloxy bedeutet vorzugsweise Acetoxy, Propionyloxy, Butyryloxy, Valeryloxy, Capronyloxy, Heptanoyloxy, Octanoyloxy, Nonanoyloxy oder Decanoyloxy; Alkoxycarbonyl bedeutet vorzugsweise Methoxycarbonyl, Ethoxycarbonyl, n-Propyloxycarbonyl, n-Butyloxycarbonyl, n-Pentyloxycarbonyl, n-Hexyloxycarbonyl, n-Heptyloxycarbonyl, n-Octyloxycarbonyl oder n-Nonyloxycarbonyl.

Bevorzugt sind ferner Verbindungen der Formel I, worin, falls n = O und gleichzeitig m = 2 ist, der Rest $R^1$ H, Alkanoyloxy oder Alkoxycarbonyl mit jeweils 1 bis 10 C-Atomen, F, Cl, Br, CN oder $CF_3$ bedeutet.

Verbindungen der Formel I sowie Ia bis Iaa mit verzweigten Alkyl-, Alkoxy-, Alkanoyloxy- oder Alkoxycarbonylgruppen können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Verzweigte Gruppen dieser Art enthalten in der Regel nicht mehr als eine Kettenverzweigung. Bevorzugte verzweigte Alkylreste sind Isopropyl, 2-Butyl (= 1-Methylpropyl), Isobutyl (= 2-Methyl-propyl) 2-Methyl-butyl, Isopentyl (= 3-Methylbutyl), 2-Methylpentyl, 3-Methylpentyl, 2-Heptyl (= 1-Methylhexyl), 2-Octyl (= 1-Methylheptyl), 2-Ethylhexyl; bevorzugte verzweigte Alkoxyreste sind Isopropyloxy, 2-Methyl-propyloxy, 2-Methyl-butyloxy, 3-Methyl-butyloxy, 2-Methyl-pentyloxy, 3-Methylpentyloxy, 1-Methyl-hexyloxy, 1-Methyl-heptyloxy, 2-Ethyl-hexyloxy, bevorzugte verzweigte Alkanoyloxyreste sind Isobutyryloxy, 2-Methylbutyryloxy, Isovaleryloxy, 2-Methylhexanoyloxy, 2-Ethylhexanoyloxy, bevorzugte verzweigte Alkoxycarbonylreste sind Isopropyloxycarbonyl, 2-Methylpropyloxycarbonyl, 2-Methyl-butyloxycarbonyl, 3-Methyl-butyloxy-carbonyl, 2-Methyl-pentyloxy-carbonyl, 3-Methyl-pentyloxy-carbonyl, 1-Methyl-hexyloxy-carbonyl, 1-Methyl-heptyloxy-carbonyl, 2-Ethyl-hexyloxy-carbonyl.

Die Verbindungen der Formel I werden im übrigen nach an sich bekannten Methoden hergestellt, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, dass man sie aus dem Reaktionsgemisch nicht isoliert, sondern sofort weiter zu den Verbindungen der Formel I umsetzt.

Die Verbindungen der Formeln II und III werden zweckmässig in Gegenwart eines inerten Lösungsmittels wie Benzol oder Toluol und/oder eines Katalysators, z.B. einer starken Säure wie Schwefelsäure, Benzol- oder p-Toluolsulfonsäure, bei Temperaturen zwischen etwa 20 und etwa 150, vorzugsweise zwischen 80 und 120 °C, miteinander umgesetzt. Als reaktionsfähige Derivate der Verbindungen der Formeln II und III eignen sich in erster Linie einfache Acetale der Formeln $R^1$–(C)$_m$–CH($OR^3$)$_2$ bzw. IIIa

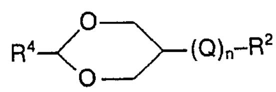

(IIIa)

worin

$R^3$ Alkyl mit 1–4 C-Atomen, zwei Reste $R^3$ zusammen auch Alkylen mit 2 oder 3 C-Atomen und

$R^4$ H, Alkyl mit 1–4 C-Atomen oder Phenyl

bedeuten.

Die Ausgangsstoffe der Formeln II und III sowie ihre reaktionsfähigen Derivate sind zum Teil bekannt, zum Teil können sie ohne Schwierigkeiten nach Standardverfahren der organischen Chemie aus literaturbekannten Verbindungen hergestellt werden. Beispielsweise sind die Aldehyde der Formel II durch Oxydation entsprechender Alkohole der Formel $R^1$–(Q)$_m$–$CH_2OH$ oder durch Reduktion entsprechender Carbonsäuren der Formel $R^1$–(Q)$_m$–COOH (oder ihrer Derivate) erhältlich, die Diole der Formel III durch Reduktion entsprechender Diester der Formel (Alkyl-$OOCCH_2$)$_2$–CH–(Q)$_n$–$R^2$.

Die erfindungsgemässen Dielektrika bestehen aus 2 bis 15, vorzugsweise 3 bis 12 Komponenten, darunter mindestens einer Verbindung der Formel I. Die anderen Bestandteile werden vorzugsweise ausgewählt aus den nematischen oder nematogenen Substanzen, insbesondere den bekannten Substanzen, aus den Klassen der Azoxybenzole, Benzylidenaniline, Biphenyle, Terphenyle, Phenyl- oder Cyclohexylbenzoate, Cyclohexancarbonsäurephenyl- oder -cyclohexylester, Phenylcyclohexane, Cyclohexylbiphenyle, Cyclohexylcyclohexane, Cyclohexylnaphthaline, 1,4-Bis-cyclohexylbenzole, 4,4-Bis-cyclohexylbiphenyle, Phenyl- oder Cyclohexyl-pyrimidine, Phenyl- oder Cyclohexyldioxane, gegebenenfalls halogenierten Stilbene, Benzylphenylether, Tolane und substituierten Zimtsäuren. Die wichtigsten als Bestandteile derartiger flüssigkristalliner Dielektrika in Frage kommenden Verbindungen lassen sich durch die Formel IV charakterisieren,

$$R^5\text{–A–G–E–}R^6 \qquad (IV)$$

worin A und E je ein carbo- oder heterocyclisches Ringsystem aus der aus 1,4-disubstituierten Benzol- und Cyclohexanringen, 4,4'-disubstituierten Biphenyl-, Phenylcyclohexan- und Cyclohexylcyclohexansystemen, 2,5-disubstituierten Pyrimidin- und 1,3-Dioxanringen, 2,6-disubstituiertem Naphthalin, Di- und Tetrahydronaphthalin, Chinazolin und Tetrahydrochinazolin gebildeten Gruppe,

| G | | |
|---|---|---|
| | –CH=CH– | –N(O)=N– |
| | –CH=CL– | –CH=N(O)– |
| | –C≡C– | $–CH_2–CH_2–$ |
| | –CO–O– | $–CH_2–O–$ |
| | –CO–S– | $–CH_2–S–$ |
| | –CH=N– | –COO–Ph–COO |

oder eine C–C-Einfachbindung, L Halogen, vorzugsweise Chlor, oder CN, und $R^5$ und $R^6$ Alkyl, Alkyloxy, Alkanoyloxy oder Alkoxycarbonyloxy mit bis zu 18, vorzugsweise bis zu 8 Kohlenstoffatomen, oder einer dieser Reste auch CN, NC, $NO_2$, $CF_3$, F, Cl oder Br bedeuten. Bei den meisten dieser Verbindungen sind $R^5$ und $R^6$ voneinander verschieden, wobei einer dieser Reste meist eine Alkyl- oder Alkoxygruppe ist. Viele solcher Substanzen oder auch Gemische davon sind im Handel erhältlich.

Die erfindungsgemässen Dielektrika enthalten etwa 0,1 bis 40, vorzugsweise 2 bis 35% einer oder mehrerer Verbindungen der Formel I.

Die Herstellung der erfindungsgemässen Dielektrika erfolgt in an sich üblicher Weise. In der Regel werden die Komponenten ineinander gelöst, zweckmässig bei erhöhter Temperatur. Wenn dabei eine Temperatur oberhalb des Klärpunkts des Hauptbestandteils gewählt wird, kann die Vollständigkeit des Lösungsvorgangs besonders leicht beobachtet werden.

Durch geeignete Zusätze können die flüssigkristallinen Dielektrika nach der Erfindung so modifiziert werden, dass sie in allen bisher bekannt gewordenen Arten von Flüssigkristallanzeigeelementen verwendet werden können.

Derartige Zusätze sind dem Fachmann bekannt und in der Literatur ausführlich beschrieben. Beispielsweise können dichroitische Farbstoffe oder Substanzen zur Veränderung der dielektrischen Anisotropie, der Viskosität, der Leitfähigkeit und/oder der Orientierung der nematischen Phasen zugesetzt werden. Derartige Substanzen sind z.B. in den DE-OS 2 209 127, 2 240 864, 2 321 632, 2 338 281, 2 450 088, 2 637 430, 2 853 728 und 2 902 177 beschrieben.

Die folgenden Beispiele sollen die Erfindung erläutern, ohne sie zu begrenzen. In den Beispielen bedeuten F. den Schmelzpunkt und K. den Klärpunkt einer flüssigkristallinen Substanz. Vor- und nachstehend bedeuten Prozentangaben Gewichtsprozent; alle Temperaturen sind in Grad Celsius angegeben.

Beispiel 1

Ein Gemisch von 2,3 g p-(trans-4-Propylcyclohexyl)-benzaldehyd (erhältlich durch Überführung der entsprechenden Säure in das Chlorid und Rosenmund-Reduktion) 1,32 g 2-Butylpropan-1,3-diol, 0,01 g p-Toluolsulfonsäure und 15 ml Toluol wird am Wasserabscheider 3 Std. gekocht, abgekühlt, mit Wasser gewaschen und eingedampft. Man erhält 2-(p-trans-4-Propylcyclohexyl-phenyl)-5-butyl-1,3-dioxan, F. 80°, K. 150°.

Beispiel 2

Analog Beispiel 1 erhält man aus den entsprechenden Aldehyden und den entsprechenden 1,3-Diolen:

2. 2-(4-Biphenylyl)-5-methyl-1,3-dioxan.
3. 2-(4'-Propyl-4-biphenylyl)-5-propyl-1,3-dioxan.
4. 2-(4'-Propyl-4-biphenylyl)-5-butyl-1,3-dioxan.
5. 2-(4'-Propyl-4-biphenylyl)-5-pentyl-1,3-dioxan.
6. 2-(4'-Butyl-4-biphenylyl)-5-propyl-1,3-dioxan.
7. 2-(4'-Butyl-4-biphenylyl)-5-butyl-1,3-dioxan.
8. 2-(4'-Butyl-4-biphenylyl)-5-pentyl-1,3-dioxan.
9. 2-(4'-Pentyl-4-biphenylyl)-5-propyl-1,3-dioxan.
10. 2-(4'-Pentyl-4-biphenylyl)-5-butyl-1,3-dioxan.
11. 2-(4'-Pentyl-4-biphenylyl)-5-pentyl-1,3-dioxan.
12. 2-(4'-Decyl-4-biphenylyl)-5-propyl-1,3-dioxan.
13. 2-(4'-Cyan-4-biphenylyl)-5-propyl-1,3-dioxan.
14. 2-(4'-Cyan-4-biphenylyl)-5-butyl-1,3-dioxan.
15. 2-(4'-Cyan-4-biphenylyl)-5-pentyl-1,3-dioxan.
16. 2-(p-trans-4-Propylcyclohexyl-phenyl)-5-propyl-1,3-dioxan.
17. 2-(p-trans-4-Propylcyclohexyl-phenyl)-5-pentyl-1,3-dioxan.
18. 2-(p-trans-4-Butylcyclohexyl-phenyl)-5-propyl-1,3-dioxan.
19. 2-(p-trans-4-Butylcyclohexyl-phenyl)-5-butyl-1,3-dioxan.
20. 2-(p-trans-4-Butylcyclohexyl-phenyl)-5-pentyl-1,3-dioxan.
21. 2-(p-trans-4-Pentylcyclohexyl-phenyl)-5-propyl-1,3-dioxan.
22. 2-(p-trans-4-Pentylcyclohexyl-phenyl)-5-butyl-1,3-dioxan.
23. 2-(p-trans-4-Pentylcyclohexyl-phenyl)-5-pentyl-1,3-dioxan.
24. 2-Phenyl-5-(trans-4-propylcyclohexyl-1,3-dioxan.
25. 2-p-Tolyl-5-(trans-4-propylcyclohexyl)-1,3-dioxan.
26. 2-p-Methoxyphenyl-5-(trans-4-propylcyclohexyl)-1,3-dioxan.
27. 2-p-Decyloxyphenyl-5-(trans-4-propylcyclohexyl)-1,3-dioxan.
28. 2-p-Formyloxyphenyl-5-(trans-4-propylcyclohexyl)-1,3-dioxan.
29. 2-p-Decanoyloxyphenyl-5-(trans-4-propylcyclohexyl)-1,3-dioxan.
30. 2-p-Methoxycarbonylphenyl-5-(trans-4-propylcyclohexyl)-1,3-dioxan.
31. 2-p-Nonyloxycarbonylphenyl-5-(trans-4-propylcyclohexyl)-1,3-dioxan.

32. 2-p-Fluorphenyl-5-(trans-4-propylcyclohexyl)-1-3,dioxan.
33. 2-p-Chlorphenyl-5-(trans-4-propylcyclohexyl)-1,3-dioxan.
34. 2-p-Bromphenyl-5-(trans-4-propylcyclohexyl)-1,3-dioxan.
35. 2-p-Trifluormethylphenyl-5-(trans-4-propylcyclohexyl)-1,3-dioxan.
36. 2-p-Cyanphenyl-5-(trans-4-propylcyclohexyl-1,3-dioxan.
37. 2-p-Cyanphenyl-5-(trans-4-butylcyclohexyl)-1,3-dioxan.
28. 2-p-Cyanophenyl-5-(trans-4-pentylcyclohexyl)-1,3-dioxan.
39. 2-(4′-Methoxy-4-biphenylyl)-5-p-propylphenyl-1,3-dioxan.
40. 2-(4′-Propyl-4-biphenylyl)-5-(trans-4-propylcyclohexyl)-1,3-dioxan.
41. 2-p-(trans-4-Propylcyclohexyl)-phenyl-5-p-propylphenyl-1,3-dioxan.
42. 2-p-(trans-4-Propylcyclohexyl)-phenyl-5-(trans-4-propylcyclohexyl)-1,3-dioxan.
43. 2-p-Cyanphenyl-5-(4′-propyl-4-biphenylyl)-1,3-dioxan.
44. 2-p-Cyanphenyl-5-(4′-butyl-4-biphenylyl-1,3-dioxan.
45. 2-p-Cyanphenyl-5-(4′-pentyl-4-biphenylyl)-1,3-dioxan.
46. 2-p-Propylphenyl-5-(p-trans-4-propylcyclohexyl-phenyl)-1,3-dioxan.
47. 2-p-Propylphenyl-5-(p-trans-4-butylcyclohexyl-phenyl)-1,3-dioxan.
48. 2-p-Propylphenyl-5-(p-trans-4-pentylcyclohexyl-phenyl)-1,3-dioxan.
49. 2-p-Butylphenyl-5-(p-trans-4-propylcyclohexyl-phenyl)-1,3-dioxan.
50. 2-p-Butylphenyl-5-(p-trans-4-butylcyclohexyl-phenyl)-1,3-dioxan.
51. 2-p-Butylphenyl-5-(p-trans-4-pentylcyclohexyl-phenyl)-1,3-dioxan.
52. 2-p-Pentylphenyl-5-(p-trans-4-propylcyclohexyl-phenyl)-1,3-dioxan.
53. 2-p-Pentylphenyl-5-(p-trans-4-butylcyclohexyl-phenyl)-1,3-dioxan.
54. 2-p-Pentylphenyl-5-(p-trans-4-pentylcyclohexyl-phenyl)-1,3-dioxan.
55. 2-p-Hexylphenyl-5-(p-trans-4-propylcyclohexyl-phenyl)-1,3-dioxan.
56. 2-p-Hexylphenyl-5-(p-trans-4-butylcyclohexyl-phenyl-1,3-dioxan.
57. 2-p-Hexylphenyl-5-(p-trans-4-pentylcyclohexyl-phenyl)-1,3-dioxan.
58. 2-p-Propyloxyphenyl-5-(p-trans-4-propylcyclohexyl-phenyl)-1,3-dioxan.
59. 2-p-Propyloxyphenyl-5-(p-trans-4-butylcyclohexyl-phenyl)-1,3-dioxan.
60. 2-p-Propyloxyphenyl-5-(p-trans-4-pentylcyclohexyl-phenyl)-1,3-dioxan.
61. 2-p-Butyloxyphenyl-5-(p-trans-4-propylcyclohexyl-phenyl)-1,3-dioxan.
62. 2-p-Butyloxyphenyl-5-(p-trans-4-butylcyclohexyl-phenyl)-1,3-dioxan.
63. 2-p-Butyloxyphenyl-5-(p-trans-4-pentylcyclohexyl-phenyl)-1,3-dioxan.
64. 2-p-Pentyloxyphenyl-5-(p-trans-4-propylcyclohexyl-phenyl)-1,3-dioxan.
65. 2-p-Pentyloxyphenyl-5-(p-trans-4-butylcyclohexyl-phenyl)-1,3-dioxan.
66. 2-p-Pentyloxyphenyl-5-(p-trans-4-pentylcyclohexyl-phenyl)-1,3-dioxan.
67. 2-p-Hexyloxyphenyl-5-(p-trans-4-propylcyclohexyl-phenyl)-1,3-dioxan.
68. 2-p-Hexyloxyphenyl-5-(p-trans-4-butylcyclohexyl-phenyl)-1,3-dioxan.
69. 2-p-Hexyloxyphenyl-5-(p-trans-4-pentylcyclohexyl-phenyl)-1,3-dioxan.
70. 2-p-Propyloxycarbonylphenyl-5-(p-trans-4-propylcyclohexyl-phenyl)-1,3-dioxan.
71. 2-p-Propyloxycarbonylphenyl-5-(p-trans-4-butylcyclohexyl-phenyl)-1,3-dioxan.
72. 2-p-Propyloxycarbonylphenyl-5-(p-trans-4-pentylcyclohexyl-phenyl)-1,3-dioxan.
73. 2-p-Butyloxycarbonylphenyl-5-(p-trans-4-propylcyclohexyl-phenyl)-1,3-dioxan.
74. 2-p-Butyloxycarbonylphenyl-5-(p-trans-4-butylcyclohexyl-phenyl)-1,3-dioxan.
75. 2-p-Butyloxycarbonylphenyl-5-(p-trans-4-pentylcyclohexyl-phenyl)-1,3-dioxan.
76. 2-p-Pentyloxycarbonylphenyl-5-(p-trans-4-propylcyclohexyl-phenyl)-1,3-dioxan.
77. 2-p-Pentyloxycarbonylphenyl-5-(p-trans-4-butylcyclohexyl-phenyl)-1,3-dioxan.
78. 2-p-Pentyloxycarbonylphenyl-5-(p-trans-4-pentylcyclohexyl-phenyl)-1,3-dioxan.
79. 2-p-Hexyloxycarbonylphenyl-5-(p-trans-4-propylcyclohexyl-phenyl)-1,3-dioxan.
80. 2-p-Hexyloxycarbonylphenyl-5-(p-trans-4-butylcyclohexyl-phenyl)-1,3-dioxan.
81. 2-p-Hexyloxycarbonylphenyl-5-(p-trans-4-pentylcyclohexyl-phenyl)-1,3-dioxan.
82. 2-p-Cyanphenyl-5-(p-trans-4-propylcyclohexyl-phenyl)-1,3-dioxan.
83. 2-p-Cyanphenyl-5-(p-trans-4-butylcyclohexyl-phenyl)-1,3-dioxan.
84. 2-p-Cyanphenyl-5-(p-trans-4-pentylcyclohexyl-phenyl)-1,3-dioxan.
85. 2-p-Cyanphenyl-5-(trans-4-p-propyloxyphenyl-cyclohexyl)-1,3-dioxan.
86. 2-p-Cyanphenyl-5-[trans-4-(trans-4-propylcyclohexyl)-cyclohexyl]-1,3-dioxan.

Es folgen Beispiele für Dielektrika mit einem Gehalt an mindestens einer Verbindung der Formel I:

Beispiel A

Ein Gemisch aus
17% p-(trans-4-Propylcyclohexyl)-benzonitril,
12% p-(trans-4-Butylcyclohexyl)-benzonitril,
24% p-(trans-4-Pentylcyclohexyl)-benzonitril,
14% p-(trans-4-Heptylcyclohexyl)-benzonitril,
15% 2-(p-trans-4-Propylcyclohexyl-phenyl)-5-butyl-1,3-dioxan und
18% 2-(p-trans-4-Propylcyclohexyl-phenyl)-5-pentyl-1,3-dioxan
zeigt F. −12°, K. 81°.

Beispiel B

Ein Gemisch aus
16% trans-1-(p-Ethoxyphenyl)-4-propylcyclohexan,

12% trans-1-(p-Butoxyphenyl)-4-propylcyclohexan,
16% p-(trans-4-Propylcyclohexyl)-benzonitril,
23% p-(trans-4-Pentylcyclohexyl)-benzonitril,
15% 2-(p-trans-4-Propylcyclohexyl-phenyl)-5-butyl-1,3-dioxan und
18% 2-(p-trans-4-Propylcyclohexyl-phenyl)-5-pentyl-1,3-dioxan
zeigt F. −10°, K. 76°.

Beispiel C
Ein Gemisch aus
8% 2-p-Cyanphenyl-5-propyl-1,3-dioxan,
10% 2-p-Cyanphenyl-5-butyl-1,3-dioxan,
8% 2-p-Cyanphenyl-5-pentyl-1,3-dioxan,
12% p-(trans-4-Propylcyclohexyl)-benzonitril,
8% p-(trans-4-Butylcyclohexyl)-benzonitril,
18% p-(trans-4-Pentylcyclohexyl)-benzonitril,
11% 4-Cyan-4'-(trans-4-pentylcyclohexyl)-biphenyl,
15% 2-(p-trans-4-Propylcyclohexyl-phenyl)-5-butyl-1,3-dioxan und
10% 2-(p-trans-4-Propylcyclohexyl-phenyl)-5-pentyl-1,3-dioxan
zeigt F. −15°, K. 88°.

**Patentansprüche**

1. 1,3-Dioxane der Formel I

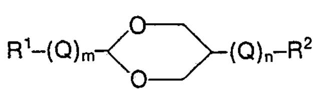 (I)

worin
$R^1$ H, Alkyl, Alkoxy, Alkanoyloxy oder Alkoxycarbonyl mit jeweils 1 bis 10 C-Atomen, F, Cl, Br, $CF_3$ oder CN,
$R^2$ Alkyl oder Alkoxy mit jeweils 1–10 C-Atomen, die Reste Q, die gleich oder verschieden sein können, 1,4-Phenylen- oder 1,4-Cyclohexylenreste und die Parameter m und n jeweils 0, 1 oder 2, ihre Summe (m + n) jedoch 2 oder 3 bedeuten, mit der Massgabe, dass im Falle m = n = 1 mindestens einer der beiden Reste Q ein 1,4-Cyclohexylenrest ist.

2. Verfahren zur Herstellung von 1,3-Dioxanen der Formel I

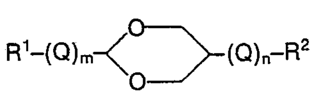 (I)

worin
$R^1$ H, Alkyl, Alkoxy, Alkanoyloxy oder Alkoxycarbonyl mit jeweils 1 bis 10 C-Atomen, F, Cl, Br, $CF_3$ oder CN,
$R^2$ Alkyl oder Alkoxy mit jeweils 1–10 C-Atomen, die Reste Q, die gleich oder verschieden sein können, 1,4-Phenylen- oder 1,4-Cyclohexylenreste und die Parameter m und n jeweils 0, 1 oder 2, ihre Summe (m + n) jedoch 2 oder 3 bedeuten, mit der Massgabe, dass im Falle m = n = 1 mindestens einer der beiden Reste Q ein 1,4-Cyclohexylenrest ist, dadurch gekennzeichnet, dass man einen Aldehyd der Formel II

$$R^1-(Q)_m-CHO \qquad (II)$$

worin
$R^1$, Q und m die angegebene Bedeutung haben oder eines seiner funktionellen Derivate mit einem Diol der Formel III

$$(HOCH_2)_2CH-(Q)_n-R^2 \qquad (III)$$

worin
$R^2$, Q und n die angegebene Bedeutung haben, umsetzt.

3. Verwendung der 1,3-Dioxane der Formel I nach Anspruch 1 als Komponenten flüssigkristalliner Dielektrika für elektrooptische Anzeigeelemente.

4. Flüssigkristallines Dielektrikum für elektrooptische Anzeigeelemente mit mindestens zwei flüssigkristallinen Komponenten, dadurch gekennzeichnet, dass mindestens eine Komponente ein 1,3-Dioxan der Formel I nach Anspruch 1 ist.

5. Elektrooptisches Anzeigeelement auf der Basis einer Flüssigkristallzelle, dadurch gekennzeichnet, dass die Flüssigkristallzelle ein Dielektrikum nach Anspruch 4 enthält.

**Claims**

1. 1,3-Dioxanes of the formula I

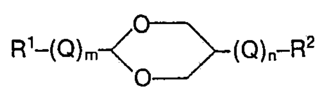 (I)

wherein
$R^1$ is H, alkyl, alkoxy, alkanoyloxy or alkoxycarbonyl with in each case 1 to 10 C atoms, F, Cl, Br, $CF_3$ or CN,
$R^2$ is alkyl or alkoxy with in each case 1–10 C atoms,
the radicals Q, which can be identical or different, are 1,4-phenylene or 1,4-cyclohexylene radicals, and the parameters m and n are in each case 0, 1 or 2, but their sum (m + n) is 2 or 3, with the proviso that in the case where m = n = 1, at least one of the two radicals Q is a 1,4-cyclohexylene radical.

2. Process for the preparation of 1,3-dioxane of the formula I

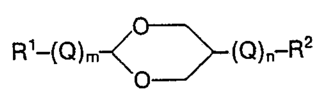 (I)

wherein
$R^1$ is H, alkyl, alkoxy, alkanoyloxy or alkoxycarbonyl with in each case 1 to 10 C atoms, F, Cl, Br, $CF_3$ or CN,
$R^2$ is alkyl or alkoxy with in each case 1–10 C atoms,
the radicals Q, which can be identical or different, are 1,4-phenylene or 1,4-cyclohexylene radicals, and the parameters m and n are in each case 0, 1 or 2, but their sum (m + n) is 2 or 3, with the proviso that in the case where m = n = 1, at least one of the two radicals Q is a 1,4-cyclohexylene radical, characterised in that an aldehyde of the formula II

$R^1$–(Q)$_m$–CHO II

wherein

$R^1$, Q and m have the meanings given, or one of its functional derivatives, is reacted with a diol of the formula III

$(HOCH_2)_2CH$–(Q)$_n$–$R^2$ III

wherein

$R^2$, Q and n have the meanings given.

3. Use of the 1,3-dioxanes of the formula I according to Claim 1 as components of liquid crystal dielectrics for electrooptical display elements.

4. Liquid crystal dielectric for electrooptical display elements with at least two liquid crystal components, characterised in that at least one component is a 1,3-dioxane of the formula I according to Claim 1.

5. Electrooptical display element based on a liquid crystal cell, characterised in that the liquid crystal cell contains a dielectric according to Claim 4.

## Revendications

1. 1,3-dioxannes de formule I

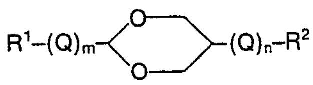 (I)

dans laquelle

$R^1$ représente H, un groupe alkyle, alcoxy, alcanoyloxy ou alcoxycarbonyle contenant chacun 1 à 10 atomes de carbone, F, Cl, Br, $CF_3$ ou CN,

$R^2$ représente un groupe alkyle ou alcoxy contenant chacun 1 à 10 atomes de carbone,
les symboles Q, qui peuvent avoir des significations identiques ou différentes, représentent des groupes 1,4-phénylène ou 1,4-cyclohexylène et les paramètres m et n sont chacun égaux à 0, 1 ou 2, leur somme (m+n) étant toutefois égale à 2 ou 3, sous réserve que dans le cas où m = n = 1, au moins des deux symboles Q représente un groupe 1,4-cyclohexylène.

2. Procédé de préparation des 1,3-dioxannes de formule I

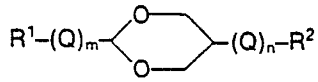 (I)

dans laquelle

$R^1$ représente H, un groupe alkyle, alcoxy, alcanoyloxy ou alcoxycarbonyle contenant chacun 1 à 10 atomes de carbone, F, Cl, Br, $CF_3$ ou CN,

$R^2$ représente un groupe alkyle ou alcoxy contenant chacun 1 à 10 atomes de carbone,
les symboles Q, qui peuvent avoir des significations identiques ou différentes, représentent des groupes 1,4-phénylène ou 1,4-cyclohexylène, et les paramètres m et n sont chacun égaux à 0, 1 ou 2, leur somme (m+n) devant toutefois être égale à 2 ou 3, sous réserve que dans le cas où m = n = 1, au moins un des deux symboles Q représente un groupe 1,4-cyclohexylène, caractérisé en ce que l'on fait réagir un aldéhyde de formule II

$R^1$–(Q)$_m$–CHO (II)

dans laquelle $R^1$, Q et m ont les significations indiquées ci-dessus,
ou l'un de ses dérivés fonctionnels,
avec un diol de formule III

$(HOCH_2)_2CH$–(Q)$_n$–$R^2$ (III)

dans laquelle $R^2$, Q et n ont les significations indiquées ci-dessus.

3. Utilisation des 1,3-dioxannes de formule I selon la revendication 1 en tant que composants de diélectriques à cristaux liquides pour éléments d'affichage électro-optiques.

4. Diélectrique à cristaux liquides pour éléments d'affichage électro-optiques à au moins deux composants à cristaux liquides, caractérisé en ce que l'un au moins des composants consiste en un 1,3-dioxanne de formule I selon la revendication 1.

5. Elément d'affichage électro-optique à base d'une cellule à cristaux liquides, caractérisé en ce que la cellule à cristaux liquides contient un diélectrique selon la revendication 4.